# EUROPEAN PATENT APPLICATION

(11) **EP 4 850 682 A1**
(43) Date of publication of application: **09.12.2026**
(21) Application number: 25749049.0
(22) Date of filing: 31.01.2025
(51) Int. Cl.: A61K 38/26, A61K 9/00, A61K 9/113, A61K 47/34, A61K 47/02, A61P 3/04, A61P 3/10

(54) **DEPOT COMPOSITION WITH CONTROLLED INITIAL RELEASE THROUGH HYDROPHOBIC ION PAIRING GELATION FOR TREATMENT OF OBESITY AND DIABETES FOR PROVIDING IMPROVED MEDICAL THERAPY OF SEMAGLUTIDE OR TIRZEPATIDE, AND METHOD FOR PREPARING SAME**

(30) Priority: 29.01.2024 KR 20240013551; 11.07.2024 KR 20240091806
(71) Applicant: TIONLAB THERAPEUTICS CO., LTD., Gyeongsangnam-do 50969 (KR)
(72) Inventor: CHOI, Go Yeong, Hwaseong-si Gyeonggi-do 18376 (KR); BAIK, Hye Jung, Seoul 06787 (KR); LIM, Duck Soo, Suwon-si Gyeonggi-do 16628 (KR)
(74) Representative: Schiweck Weinzierl Koch Patentanwälte Partnerschaft mbB
(86) International application number: PCT/KR2025/001575
(87) International publication number: WO 2025/165146

(57) **Abstract**

The present invention relates to a depot composition comprising calcium chloride inside microspheres. The depot composition according to the present invention can control excessive release of an active ingredient contained inside microspheres at the initial stage of depot injection by using calcium chlorides as a cationic salt. In addition, the composition has excellent suspendability and a significantly small amount of residual solvent, and thus even when administered as an injection by a user, the effect of the active ingredient can be uniformly and continuously obtained. In addition, improved medical therapy with semaglutide can be provided by adjusting the dose of semaglutide or tirzepatide by means of the depot formulation.

## Description

### [Technical Field]

The present invention provides improved medical therapy with semaglutide or tirzepatide.

Further, the present invention relates to a depot composition, and the depot composition controls the initial burst of microspheres through hydrophobic ion pairing gelation and also significantly improves the amount of residual solvent removed. Therefore, the depot composition extends the period of administration without repeated administration of the depot composition and therefore has no sudden side effects even when an excessive amount of a drug is administered, and provides high user convenience and excellent therapeutic effects through excellent anti-diabetic and anti-obesity effects.

### [Background Art]

A depot preparation is a formulation that prolongs the effectiveness of medicine, and is a formulation that can be primarily used when administering hormones or the like over a long period of time. A depot preparation, which is a sustained-release formulation, has an advantage in that the number of injections to a patient can be minimized. However, it is not easy for a depot preparation to implement constant and sustained release of a drug over the entire release period after injection. In particular, in the case of a high initial burst rate, in which an excessive amount of drug is released from the depot preparation at the beginning of injection, side effects due to the excess drug may occur and the sustained efficacy of the drug may decrease during the specified period. Therefore, it is very important to control the initial burst in a depot preparation.

GLP-1 agonists such as semaglutide were first developed to lower blood glucose in diabetic patients, and since the drugs have also been confirmed to be effective in reducing body weight in obese patients, they are used to treat diabetes and obesity. Semaglutide is an analogue of incretin hormones secreted in the intestines, that increases the amount of insulin secreted after a meal and allows food to move more slowly from the stomach to the small intestine by increasing the time it takes for the contents of the stomach to be released. Further, semaglutide can act on the central nervous system, thereby helping to lower blood glucose and reduce body weight through various actions of suppressing appetite.

Meanwhile, tirzepatide is a dual agonist that acts as a GLP-1 receptor agonist and a GIP receptor agonist, and has effects of controlling blood glucose and improving obesity, like semaglutide. Glucose-dependent insulinotropic polypeptide (GIP) is an incretin hormone, and is secreted from the small intestine in response to food, like GLP-1. Although this substance was previously recognized as an obesity hormone because it induces glucagon secretion along with insulin secretion, it counteracts the glucagon secretion caused by GIP while acting together with GLP-1, and actually has the effect of reducing fat accumulation. Therefore, tirzepatide, a dual agonist of the GLP-1/GIP receptor, may have effects of improving blood glucose and treating obesity similar to semaglutide.

However, when the body is exposed to excessive amounts of GLP-1 and GLP-1/GIP receptor agonists, side effects such as nausea, diarrhea, increased heart rate, or headache may occur. Therefore, controlling the initial burst of a depot preparation containing GLP-1 and GLP/GIP receptor agonists as active ingredients is an increasingly important challenge. Ozempic (for the treatment of diabetes) and Wegovy (for the treatment of obesity), which are already approved semaglutide-containing products, and Mounjaro (for the treatment of diabetes) and Zepbound (for the treatment of obesity) begin with low doses and are gradually increased to higher doses after the body has become accustomed to the drugs. This is due to side effects that occur when the body is exposed to high doses of the drugs, highlighting the importance of controlling initial exposure.

Meanwhile, Japanese Patent No. 5681626 discloses that a sustained-release preparation including microspheres in which sterol-containing lipid components and polylactide-co-glycolide (PLGA) polymers are combined and a phospholipid component can control the release rate. However, lipid components have disadvantages of failing to show sufficient effects in suppressing the initial burst and causing the properties of the microspheres to deteriorate due to their strong hydrophobic properties or reducing their suspendability due to the aggregation of the microspheres.

Further, according to Korean Patent Application No. 10-2011-0145945, a novel synthetic polymer is provided, in which polylactide-co-glycolide (PLGA) polymers that form microspheres are synthesized with polyvalent ions, and the polyvalent ions are bonded to the polymer. However, the novel synthetic polymer is required to be subjected to toxicity testing for use in actual clinical settings, making it difficult to obtain approval. Therefore, there are limitations to applying the polymer to depot preparations and using it in practice.

Furthermore, Korean Patent No. 10-409413 provides a method for preparing microspheres by an underwater drying method, and then heating and drying the microspheres at a temperature equal to or higher than the glass transition temperature (Tg) of a biodegradable polymer, thereby significantly suppressing initial excessive release and minimizing the use of organic solvents. However, this preparation method has a disadvantage in that a physiologically active substance may be denatured by heat.

As described above, since conventional sustained-release microspheres have limitations in providing a depot preparation with a sufficiently controlled initial burst, there remains a need to develop a depot composition having the properties of being able to control initial burst and maintain the drug for a desired period of time.

In addition, in the prevention or treatment of obesity or diabetes, there is a need for improved medical therapy to alleviate gastrointestinal disorders, particularly vomiting, abdominal pain, nausea, and the like, which are abnormal reactions to semaglutide or tirzepatide.

### [Disclosure]

### [Technical Problem]

The present invention has been made in an effort to solve the aforementioned problems, and has an object to provide a depot composition that suppresses excessive initial burst and remarkably reduces the amount of residual solvent, and a method for preparing the same.

Furthermore, the present invention has an object to provide improved medical therapy with semaglutide and/or tirzepatide, which can exhibit long-term therapeutic effects by including semaglutide or a pharmaceutically acceptable salt thereof; or tirzepatide or a pharmaceutically acceptable salt thereof.

As a result of making efforts to develop a depot composition capable of controlling the excessive initial burst of a drug, the present inventors confirmed that the initial burst of a drug can be suppressed by containing calcium chloride in the water phase layer of microspheres. Further, it was confirmed that containing a plasticizer in the oil phase layer of microspheres can remarkably increase the amount of residual solvent removed. It was confirmed that such microspheres, when used as depot preparations, can be technologically controlled to prevent the initial burst of a drug while containing a large amount of the drug during a target period, and that they possess preparation characteristics with excellent long-term therapeutic effects, thereby completing the present invention.

### [Technical Solution]

The present invention provides a pharmaceutical composition for use in preventing or treating obesity or diabetes, including semaglutide or a pharmaceutically acceptable salt thereof; or tirzepatide or a pharmaceutically acceptable salt thereof, as an active ingredient,
wherein the dosage of a GLP-1 receptor agonist for preventing or treating obesity is 12 to 96 mg/4 weeks, and
the dosage of a GLP-1 receptor agonist for preventing or treating diabetes is 4 to 80 mg/4 weeks.

In addition, the present invention provides a depot composition including microspheres in an O/W2 form, including an oil phase layer (O layer) and a second water phase layer (W2 layer), or microspheres in a W1/O/W2 form, including a first water phase layer (W1 layer), an oil phase layer (O layer), and a second water phase layer (W2 layer),
wherein the oil phase layer (O layer) in the microspheres in the O/W2 form or the first water phase layer (W1 layer) in the microspheres in the W1/O/W2 form includes semaglutide or a pharmaceutically acceptable salt thereof; or tirzepatide or a pharmaceutically acceptable salt thereof, and
in the microspheres in the O/W2 form or microspheres in the W1/O/W2 form, the oil phase layer (O layer) includes a biodegradable polymer, and the second water phase layer (W2 layer) includes calcium chloride.

Furthermore, the present invention provides a method for preparing a depot composition, the method including: preparing an oil phase layer (O layer) solution or mixing a first water phase layer (W1 layer) solution with the oil phase layer (O layer) solution to prepare a W1/O emulsion; and
introducing the oil phase layer (O layer) solution or W1/O emulsion into a second water phase (W2 layer) layer solution to prepare an O/W2 emulsion or W1/O/W2 emulsion,
wherein the oil phase layer (O layer) solution in the O/W2 emulsion, or the water phase layer (W1 layer) solution in the W1/O/W2 emulsion includes semaglutide or a pharmaceutically acceptable salt thereof; or tirzepatide or a pharmaceutically acceptable salt thereof, and
the oil phase layer (O layer) solution in the O/W2 emulsion or W1/O/W2 emulsion includes a biodegradable polymer, and the second water phase layer (W2 layer) solution includes calcium chloride.

### [Advantageous Effects]

The microspheres of the depot composition of the present invention can control excessive release of an active ingredient contained inside the microspheres at the initial stage of injection of a depot preparation by including calcium chloride in the water phase layer. In addition, the composition has excellent suspendability and a significantly small amount of residual solvent, and thus even when administered as an injection by a user, the effect of the active ingredient can be uniformly, safely, and continuously obtained.

In addition, improved medical therapy with semaglutide and/or tirzepatide can be provided by adjusting the dose of semaglutide and/or tirzepatide by means of the depot formulation.

### [Description of Drawings]

FIGS. 1A to 1C are SEM photographs of microspheres prepared according to Examples 1-3 (A), 1-9 (B), and 1-13 (C) of the present invention.
FIG. 2 is an SEM photograph of microspheres prepared according to Comparative Example 1-1 of the present invention.
FIG. 3 is an SEM photograph of microspheres prepared according to Comparative Example 2-7 of the present invention.
FIG. 4A shows the animal PK profiles of microspheres prepared according to Comparative Example 1-1, Examples 1-3 and 1-5, and Comparative Example 2-7 of the present invention. In addition, FIG. 4B shows the animal PK profiles of microspheres prepared according to Comparative Example 1-6 and Example 2-6.
FIGS. 5A to 5E show the DSC results for 504H polymer (A) and microspheres prepared according to Comparative Examples 1-1 (B) and 2-7 (C) and Examples 1-3 (D) and 1-6 (E) of the present invention.
FIGS. 6A to 6E show the DSC results for 504H polymer (A) and microspheres prepared according to Comparative Examples 1-1 (B) and 2-7 (C) and Examples 1-3 (D) and 1-6 (E) of the present invention.
FIGS. 7A and 7B show the glucose-lowering effect (A) and glycated hemoglobin lowering effect (B) of a semaglutide depot preparation in diabetic animal models, according to the dose.
FIG. 8 shows the body weight-loss effect of a semaglutide depot preparation in an obesity-inducing animal model according to the dose.

### [Best Mode]

Hereinafter, the configuration of the present invention will be described in detail.

In the present invention, unless otherwise specified, numerical limitations indicate "or more" or "or less."

The present invention provides a pharmaceutical composition for use in prevention or treatment of obesity or diabetes, including a GLP-1 receptor agonist or a GLP-1/GIP receptor dual agonist as an active ingredient.

In the present invention, the GLP-1 receptor agonist is semaglutide or a pharmaceutically acceptable salt thereof, and the GLP-1/GIP receptor dual agonist is tirzepatide or a pharmaceutically acceptable salt thereof.

In the present invention, semaglutide or tirzepatide may be used for the prevention or treatment of obesity or diabetes as a GLP-1 receptor agonist involved in the action of glucagon-like peptide-1 (GLP-1), which is a hormone that serves to lower blood glucose. Among these, tirzepatide is a glucose-dependent insulin-secreting polypeptide, that is, a GIP hormone, and may be similarly used as an agent for the prevention or treatment of obesity or diabetes, when used in combination with GLP-1.

In an embodiment, semaglutide or a pharmaceutically acceptable salt thereof; or tirzepatide or a pharmaceutically acceptable salt thereof may each be selected from the group consisting of an acetate, benzoate, hydroxynaphthoate, napadisilate, or pamoate.

In an embodiment, the suitable dosage of semaglutide or a pharmaceutically acceptable salt thereof; or tirzepatide or a pharmaceutically acceptable salt thereof may vary widely depending on the patient's body weight, age, sex, health condition, diet, administration time, administration method, excretion rate, severity of a disease, and the like. For example, the daily dosage of semaglutide or a pharmaceutically acceptable salt thereof; or tirzepatide or a pharmaceutically acceptable salt thereof may be 0.357 to 3.428 mg, preferably 1 to 1.714 mg, for the treatment of obesity, and 0.142 to 2.857 mg, preferably 0.428 to 1.142 mg, for the treatment of diabetes, based on semaglutide or tirzepatide.

In an embodiment, the suitable administration interval for semaglutide or a pharmaceutically acceptable salt thereof; or tirzepatide or a pharmaceutically acceptable salt thereof may be determined according to the aforementioned dosage. For example, the semaglutide or a pharmaceutically acceptable salt thereof, or tirzepatide or a pharmaceutically acceptable salt thereof of the present invention may each be administered once a month to once every six months, preferably once a month, once every two months, once every three months, or once every six months, and most preferably once a month.

When the pharmaceutical composition according to the present invention is used for the prevention or treatment of obesity, the dosage of semaglutide or a pharmaceutically acceptable salt thereof; or tirzepatide or a pharmaceutically acceptable salt thereof is 12 to 96 mg/4 weeks for each. Furthermore, when used for the prevention or treatment of diabetes, the dosage of semaglutide or a pharmaceutically acceptable salt thereof; or tirzepatide or a pharmaceutically acceptable salt thereof is 4 to 80 mg/4 weeks for each.

In the present invention, obesity may be prevented or treated without side effects by administering 12 to 96 mg of each of semaglutide or a pharmaceutically acceptable salt thereof; or tirzepatide or a pharmaceutically acceptable salt thereof, once every four weeks. In this case, side effects are safety incidents such as gastrointestinal dysfunction, and the gastrointestinal dysfunctions may be symptoms selected from the group consisting of nausea, vomiting, diarrhea, and constipation.

In an embodiment, in the prevention or treatment of obesity, the dosage of semaglutide or a pharmaceutically acceptable salt thereof; or tirzepatide or a pharmaceutically acceptable salt thereof may be specifically 12 to 96 mg/4 weeks, 15 to 86 mg/4 weeks, 19 to 77 mg/4 weeks, 24 to 60 mg/4 weeks, or 28 to 48 mg/4 weeks for each.

Further, in the present invention, diabetes may be prevented or treated without side effects by administering 4 to 80 mg/4 weeks of each of semaglutide or a pharmaceutically acceptable salt thereof; or tirzepatide or a pharmaceutically acceptable salt thereof, once every four weeks.

In an embodiment, in the prevention or treatment of diabetes, the dosage of semaglutide or a pharmaceutically acceptable salt thereof; or tirzepatide or a pharmaceutically acceptable salt thereof may each be specifically 4 to 80 mg/4 weeks, 6 to 64 mg/4 weeks, 8 to 48 mg/4 weeks, 10 to 40 mg/4 weeks, or 12 to 32 mg/4 weeks.

In an embodiment, the pharmaceutical composition according to the present invention may be administered to a subject as a preparation of a formulation for parenteral administration.

Specifically, the pharmaceutical composition of the present invention may be formulated into various forms of parenteral administration formulations, such as microneedle formulations and depot formulations. These pharmaceutical compositions may be administered intramuscularly, transdermally, intravenously, and/or subcutaneously.

In an embodiment, the semaglutide or a pharmaceutically acceptable salt thereof, or tirzepatide or a pharmaceutically acceptable salt thereof, according to the present invention, may be administered to a subject as a depot composition formulation.

The depot composition corresponds to a sustained-release preparation, that is, a formulation capable of releasing a drug for a long period of time. The sustained-release preparation may gradually release an internal drug to a desired level for a certain period of time, and the depot composition may control the initial burst, which is the excessive release of the internal drug at the initial stage of injection.

When the body is exposed to an excessive amount of semaglutide or tirzepatide, symptoms such as vomiting, nausea, hypoglycemia and headaches may occur. Therefore, when semaglutide or tirzepatide is used as an active ingredient of the depot composition, it is very important to prevent the active ingredient from being released in an excessive amount from microspheres of the depot composition.

In the present invention, the initial burst control effect may be secured using a depot composition to be described below. That is, since the dosage of semaglutide or tirzepatide can be adjusted, new therapy with the above-described semaglutide or tirzepatide may be provided.

In an embodiment, when formulated into a depot composition, the content of semaglutide or a pharmaceutically acceptable salt thereof; or tirzepatide or a pharmaceutically acceptable salt thereof may each be 5 to 25 wt% based on the total weight of the formulation.

In an embodiment, the pharmaceutical compositions of the present invention may exhibit an AUCₜ/Cₘₐₓ of 100 hours or longer for semaglutide or tirzepatide when administered to an individual.

In the present invention, AUC refers to the area under the plasma concentration-time curve which is a measure of total bioavailability; AUCₜ (AUCₗₐₛₜ) refers to the area under the plasma concentration-time curve from time zero to the measurement time; and Cₘₐₓ (maximum blood concentration) refers to the highest blood concentration of the drug when administered *in vivo*. Further, all of these values are pharmacokinetic parameters for a single dose.

By exhibiting the AUCₜ/Cₘₐₓ values described above, the pharmaceutical combination according to the present invention may prevent an excessively high blood concentration of the drug during administration and maintain a constant level while being therapeutically effective. This may remarkably reduce the side effects commonly associated with incretin hormones (GLP-1 receptor agonists) of semaglutide or tirzepatide, for example, gastrointestinal dysfunction side effects such as nausea, vomiting, constipation, and diarrhea, as well as side effects such as insomnia, dizziness, dysgeusia, lethargy, cholelithiasis, cholecystitis, pancreatitis, dehydration, tachycardia, or acute kidney injury.

The present invention also provides a depot composition, wherein the depot composition includes microspheres including semaglutide or a pharmaceutically acceptable salt thereof; or tirzepatide or a pharmaceutically acceptable salt thereof as an active ingredient.

The microspheres may be microspheres in an O/W2 form, including an oil phase layer (O layer) and a second water phase layer (W2 layer), or microspheres in a W1/O/W2 form, including a first water phase layer (W1 layer), an oil phase layer (O layer), and a second water phase layer (W2 layer).

By including microspheres in an O/W2 form or microspheres in a W1/O/W2 form, the depot composition of the present invention has an advantage in that initial burst is controlled and suspendability is excellent.

In the present specification, for convenience of description regarding two or more water phase layers or water phase layer solutions, a water phase layer present in the oil phase layer of the microsphere or a water phase layer solution for forming the water phase layer may be referred to as a first water phase layer (W1 layer) or a first water phase layer solution, and a water phase layer formed on the outer periphery of the oil phase layer of the microsphere or a water phase layer solution for forming the water phase layer may be referred to as a second water phase layer (W2 layer) or a second water phase layer solution. In this case, the water phase layer (W layer) or water phase layer solution in the microspheres in an O/W form is understood to have a structure corresponding to a second water phase layer (W2 layer) or second water phase layer solution in the microspheres in a W1/O/W2 form. Therefore, the O/W emulsion may also be referred to as an O/W2 emulsion in the present invention. In the following description, the reference to the second water phase layer (W2 layer) or second water phase layer solution should be understood as corresponding to the water phase layer or water phase layer solution for the microspheres in the O/W form.

In the present invention, the oil phase layer (O layer) in the microspheres in the O/W2 form or the first water phase layer (W1 layer) in the microspheres in the W1/O/W2 form includes an active ingredient. The active ingredient may include semaglutide or a pharmaceutically acceptable salt thereof; or tirzepatide or a pharmaceutically acceptable salt thereof, as an API. The depot composition according to the present invention may be administered into the body and function to release the active ingredient into the body for a certain period of time. The active ingredient is included in the microspheres to be protected from the microspheres, and may remain in the body for a certain period of time.

In an embodiment, the content of semaglutide or a pharmaceutically acceptable salt thereof; or tirzepatide or a pharmaceutically acceptable salt thereof may each be 5 to 25 wt% based on the total weight of the microspheres.

In the present invention, the oil phase layer (O layer) in the microspheres in the O/W2 form or the oil phase layer (O layer) in the microspheres in the W1/O/W2 form includes a biodegradable polymer. The biodegradable polymer has the characteristics of being degraded *in vivo*, and forms microspheres in the present invention, and as a polymer is gradually degraded *in vivo*, the active ingredient included therein may be gradually released. That is, the biodegradable polymer may serve to protect the drug therein during the drug release period and control the drug release over a long period of time.

Biodegradable polymers approved for use in injectable preparations by the drug safety authority of each country may be used without limitation, and may include, for example, a polymer selected from the group consisting of polylactic acid (PLA), polyglycolide (PGA), and polylactide-co-glycolide (PLGA).

The biodegradable polymer may have an inherent viscosity of 0.35 to 0.65 dL/g, or 0.50 to 0.55 dL/g. When the inherent viscosity is less than 0.35 dL/g, the drug may be released faster than over a desired period, whereas when the inherent viscosity is more than 0.65 dL/g, the drug may be released slower than within the desired period, making it difficult to obtain a sufficient drug effect. This inherent viscosity may be measured by a viscosity measuring method for polylactide-co-glycolide (PLGA) provided by the manufacturer.

A content of the biodegradable polymer may be 75 to 95 wt% based on the total weight of the microspheres.

In the present invention, the oil phase layer (O layer) in the microspheres in the O/W2 form or the oil phase layer (O layer) in the microspheres in the W1/O/W2 form may further include a plasticizer.

Plasticizers are used to improve the flexibility and brittleness of biodegradable polymers. The plasticizer helps an organic solvent to rapidly escape into the water phase and be removed before the polymer is solidified during the process of preparing microspheres by the water drying method and then removing the organic solvent by heating and drying to vaporize the organic solvent. Therefore, the amount of organic solvent remaining in the final microspheres may be minimized.

In an embodiment, plasticizers that have been approved for use as pharmaceutical additives by the drug safety authority of each country may be used without limitation. For example, one or more plasticizers may be selected from the group consisting of glycerin (glycerol), concentrated glycerin, sorbitol, propylene glycol, a poloxamer-based polymer, a phthalate polymer such as dimethyl-, diethyl-, and dibutyl phthalates, a citrate derivative such as tributyl-, triethyl-, or acetyl citrate, triacetin, and castor oil and used. The poloxamer may be Poloxamer 188, Poloxamer 407, and the like, and the citrate derivative may be triethyl citrate (TEC), and the like.

In an embodiment, the plasticizer may be included at a concentration of 0.1 to 10.0% (w/v) based on the oil phase layer (O layer). Specifically, when triethyl citrate is used as a plasticizer, the concentration may be 0.5 to 10% (w/v) or 1 to 8% (w/v) based on the oil phase layer (O layer), and when a poloxamer is used, the concentration may be 0.1 to 5.0% (w/v) or 0.1 to 3.0% (w/v) based on the oil phase layer (O layer).

In the present invention, the second water phase layer (W2 layer) in the microspheres in the O/W form or the microspheres in the W1/O/W2 form includes calcium chloride.

In general, microspheres are prepared in a homogenization process, and during the process of vaporizing the solvent used to dissolve the polymer, the solvent is scattered, and the microspheres are solidified. In the present invention, during the solidification process, calcium chloride may be brought into contact with the polymer on the surface of the microspheres, and a gelation phenomenon may occur at the interface due to hydrophobic ion pairing, thereby forming microspheres while coating the surfaces of the microspheres. Through this, the calcium chloride may control the initial burst of the active ingredient.

The calcium chloride may be included at a concentration of 0.1 to 5.0% (w/v), 0.1 to 4.0% (w/v), 0.1 to 3.0% (w/v), or 0.1 to 1.5% (w/v) based on the second water phase layer (W2 layer).

The depot composition of the present invention may further include a pharmaceutically acceptable carrier or excipient, and the like. However, preferably, the depot composition of the present invention may not include a stabilizer, a pH adjusting agent, and an oxidizing agent.

In addition, the present invention relates to a method for preparing the above-described depot composition.

The depot composition according to the present invention may be prepared by a single emulsification method of producing an O/W emulsion by adding an oil phase solution to a water phase solution and emulsifying the resulting mixture. In addition, the depot composition according to the present invention may be prepared by a double emulsification method of producing a W/O/W emulsion by adding a W/O emulsion to a water phase solution and emulsifying the resulting mixture.

In an embodiment, a depot composition may be prepared by preparing an oil phase layer (Oil phase: O layer) solution or mixing a first water phase layer (Water phase 1: W1 layer) solution with the oil phase layer (O layer) solution to prepare a W1/O emulsion (hereinafter, step S1); and

introducing the oil phase solution or W1/O emulsion into a second water phase layer (Water phase 2: W2 layer) solution to prepare an O/W2 emulsion or W1/O/W2 emulsion (hereinafter, step S2).

Furthermore, the preparation method of the present invention may further include vaporizing the prepared O/W2 emulsion or W1/O/W2 emulsion by heating and drying. The steps may be carried out below the glass transition temperature (Tg) of the biodegradable polymer when a plasticizer to be described below is used.

Further, the preparation method of the present invention may further include drying and/or filtering the prepared O/W2 emulsion or W1/O/W2 emulsion and then centrifuging the emulsion to recover microspheres.

In the present invention, step S1 is a step of preparing an oil phase solution or preparing a W1/O emulsion.

In an embodiment, the oil phase solution may include a fat-soluble solvent. As the fat-soluble solvent, any pharmaceutically acceptable carrier or solvent typically used in the technical field of the present invention may be used. For example, dichloromethane may be used as the fat-soluble solvent, but the fat-soluble solvent is not limited thereto.

In an embodiment, as the solvent of the first water phase layer solution, any pharmaceutically acceptable carrier or solvent typically used in the technical field of the present invention may be used. For example, polyvinyl alcohol (PVA) or sodium acetate may be used, but the solvent is not limited thereto.

In an embodiment, the first water phase solution in the oil phase solution or W1/O emulsion includes an active ingredient, and the active ingredient may be semaglutide or a pharmaceutically acceptable salt thereof, or tirzepatide or a pharmaceutically acceptable salt thereof as a GLP-1 receptor agonist. The content of the GLP-1 receptor agonist may be 5 to 25 wt%, and specifically, the content of semaglutide or a pharmaceutically acceptable salt thereof; or tirzepatide or a pharmaceutically acceptable salt thereof may each be 5 to 25 wt%.

In an embodiment, the oil phase solution includes a biodegradable polymer.

Biodegradable polymers approved for use in injectable preparations by the drug safety authority of each country may be used without limitation, and for example, a polymer selected from the group consisting of polylactic acid (PLA), polyglycolide (PGA), and polylactide-co-glycolide (PLGA) may be used.

A content of the biodegradable polymer may be 75 to 95 wt% based on the total weight of the microspheres.

In the present invention, the oil phase solution may further include a plasticizer.

As the plasticizer, for example, it is possible to use one or more plasticizers selected from the group consisting of glycerin (glycerol), concentrated glycerin, sorbitol, propylene glycol, a poloxamer-based polymer, a phthalate polymer such as dimethyl-, diethyl-, and dibutyl phthalates, a citrate derivative such as tributyl-, triethyl-, or acetyl citrate, triacetin, and castor oil and used. The poloxamer may be Poloxamer 188, Poloxamer 407, and the like, and the citrate derivative may be triethyl citrate (TEC), and the like.

In an embodiment, the plasticizer may be included at a concentration of 0.1 to 10.0% (w/v) based on the entire oil phase layer solution. Specifically, when triethyl citrate is used as a plasticizer, the concentration may be 0.5 to 10.0% (w/v) or 1 to 8% (w/v) based on the oil phase layer solution, and when a poloxamer is used, the concentration may be 0.1 to 5.0% (w/v) or 0.1 to 3.0% (w/v) based on the oil phase layer solution.

In an embodiment, the W1/O emulsion may be prepared by mixing the oil phase with the first water phase layer solution and stirring the resulting mixture with a homogenizer. The stirring speed and time may differ depending on the emulsion forming conditions and the amount of sample.

In the present invention, step S2 is a step of introducing the oil phase solution or W1/O emulsion prepared/produced in step S1 to a second water phase solution to produce an O/W2 emulsion or W1/O/W2 emulsion.

In an embodiment, as the solvent of the second water phase layer solution, any pharmaceutically acceptable carrier or solvent typically used in the technical field of the present invention may be used. For example, polyvinyl alcohol (PVA) or sodium acetate may be used, but the solvent is not limited thereto. The second water phase solution may further include an osmotic pressure adjusting agent to adjust the osmotic pressure of the solution during the emulsion preparation process.

In addition, the solvent may further include methylcellulose, polyvinylpyrrolidone, carboxymethylcellulose, lecithin, gelatin, polyoxyethylene, polyoxyethylene sorbitan fatty acid esters, polyoxyethylene castor oil derivatives, and mixtures thereof.

In an embodiment, the second water phase solution includes calcium chloride. The calcium chloride may be effectively used as a depot preparation by significantly improving the suspendability of the depot composition along with the initial burst suppression effect of suppressing the excessive release of the active ingredient in the microspheres.

The calcium chloride may be included at a concentration of 0.1 to 5.0% (w/v), 0.1 to 4.0% (w/v), 0.1 to 3.0% (w/v), or 0.1 to 1.5% (w/v) based on the entire second water phase layer solution.

In an embodiment, the O/W2 emulsion or W1/O/W2 emulsion may be prepared by stirring with a homogenizer. The stirring speed and time may differ depending on the emulsion forming conditions and the amount of sample.

The benefits and features of the present invention, and the methods of achieving the benefits and features will become apparent with reference to embodiments to be described below in detail. However, the present invention is not limited to the exemplary embodiments to be disclosed below, and may be implemented in various other forms, the present exemplary embodiments are only provided to render the disclosure of the present invention complete and fully represent the scope of the invention to a person with ordinary skill in the technical field to which the present invention pertains, and the present invention will be defined only by the scope of the claims.

### [Modes of the Invention]

### Examples

### [Examples 1 and 2] Preparation of microspheres including calcium chloride

In Example 1, 1 g of semaglutide was dissolved as the API in 4 mL of water, and in Example 2, tirzepatide was dissolved as the API in 4 mL of water to prepare a first water phase layer (W1 layer) solution. The polymers in the amounts (g) according to Tables 2 and 3 were dissolved in 60 mL of dichloromethane (Honeywell) to prepare oil phase layer (O layer) solutions. A W1/O emulsion was prepared by mixing the W1 layer solution and the O layer solution and stirring the resulting mixture with a homogenizer at 8,000 rpm for 2 minutes.

Next, calcium chloride (CaCl₂) at the concentrations shown in Tables 2 and 3 was dissolved in 5 L of a 1 wt% polyvinyl alcohol (PVA; Gohsenol EG-40PW, Nippon Gohsei) solution to prepare a second water phase (W2 layer) solution. A W1/O/W2 emulsion was prepared by stirring at 8,000 rpm with a homogenizer while injecting the W1/O emulsion into the W2 layer solution at a rate of 18 ml/min.

The prepared W1/O/W2 emulsion was warmed from 15°C to 40°C for 1 hour, and then maintained for 3 hours to vaporize it. Thereafter, the emulsion was cooled to 25°C. After filtering through a sieve with a mesh size of 75 µm, the microspheres were recovered by centrifugation. After the recovered microspheres were redispersed in distilled water, the surfaces of the microspheres were washed three times by centrifugation. The washed microspheres were freeze-dried to finally obtain drug-loaded microspheres.

Hereinafter, according to the type of API used, the microspheres may be expressed as semaglutide microspheres (depot) when semaglutide (Example 1) is used, and as tirzepatide microspheres (depot) when tirzepatide (Example 2) is used.

### [Comparative Example 1] Preparation of microspheres not including calcium chloride

Microspheres were prepared in the same manner as in Examples 1 and 2, except that calcium chloride was not used. (Table 1)

The main components of the microspheres prepared in Examples 1 and 2 and Comparative Example 1 are shown in Tables 1, 2, and 3.

**[Table 1]**

| Classification | W1 phase (g) | O phase (g) | | W2 phase (%(w/v)) |
|---|---|---|---|---|
| | Amount / API | Type of polymer | Amount of polymer | Concentration of CaCl₂ added |
| Comparative Example 1-1 | 1 /Semaglutide | PLGA504H | 9 | 0.00 |
| Comparative Example 1-2 | 1 /Semaglutide | PLGA504H | 4 | 0.00 |
| Comparative Example 1-3 | 1 /Semaglutide | PLGA 502H | 4 | 0.00 |
| Comparative Example 1-4 | 1 /Semaglutide | PLGA 752H | 4 | 0.00 |
| Comparative Example 1-5 | 1 /Semaglutide | PLA 202H | 4 | 0.00 |
| Comparative Example 1-6 | 1 /Tirzepatide | PLGA504H | 9 | 0.00 |

**[Table 2]**

| Classification | W1 phase (g) | O phase (g) | | W2 phase (%(w/v)) |
|---|---|---|---|---|
| | Amount / API | Type of polymer | Amount of polymer | Concentration of CaCl₂ added |
| Example 1-1 | 1 /Semaglutide | PLGA 504H | 9 | 0.25 |
| Example 1-2 | 1 /Semaglutide | PLGA 504H | 9 | 0.50 |
| Example 1-3 | 1 /Semaglutide | PLGA 504H | 9 | 0.75 |
| Example 1-4 | 1 /Semaglutide | PLGA 504H | 9 | 1.00 |
| Example 1-5 | 1 /Semaglutide | PLGA 504H | 9 | 1.25 |
| Example 1-6 | 1 /Semaglutide | PLGA 504H | 9 | 1.50 |
| Example 1-7 | 1 /Semaglutide | PLGA 504H | 9 | 3.00 |
| Example 1-8 | 1 /Semaglutide | PLGA 504H | 6 | 0.25 |
| Example 1-9 | 1 /Semaglutide | PLGA 504H | 6 | 0.50 |
| Example 1-10 | 1 /Semaglutide | PLGA 504H | 6 | 0.75 |
| Example 1-11 | 1 /Semaglutide | PLGA 504H | 4 | 0.25 |
| Example 1-12 | 1 /Semaglutide | PLGA 504H | 4 | 0.50 |
| Example 1-13 | 1 /Semaglutide | PLGA 504H | 4 | 0.75 |
| Example 1-14 | 1 /Semaglutide | PLGA 504H | 4 | 1.50 |
| Example 1-15 | 1 /Semaglutide | PLGA 502H | 4 | 0.50 |
| Example 1-16 | 1 /Semaglutide | PLGA 752H | 4 | 0.50 |
| Example 1-17 | 1 /Semaglutide | PLA 202H | 4 | 0.50 |
| Example 1-18 | 1 /Semaglutide | PLA 202S | 4 | 0.50 |
| Example 1-19 | 1 /Semaglutide | PLGA 504 H : 502 H = 2 : 1 | 4 | 1.50 |
| Example 1-20 | 1 /Semaglutide | PLGA 504 H : 752 H = 1 : 1 | 4 | 0.50 |

**[Table 3]**

| Classification | W1 phase (g) | O phase (g) | | W2 phase (%(w/v)) |
|---|---|---|---|---|
| | Amount / API | Type of polymer | Amount of polymer | Concentration of CaCl₂ added |
| Example 2-1 | 1 /Tirzepatide | PLGA 504H | 4 | 0.50 |
| Example 2-2 | 1 /Tirzepatide | PLGA 504H | 4 | 1.50 |
| Example 2-3 | 1 /Tirzepatide | PLGA 504H | 6 | 0.50 |
| Example 2-4 | 1 /Tirzepatide | PLGA 504H | 6 | 1.50 |
| Example 2-5 | 1 /Tirzepatide | PLGA 504H | 9 | 0.50 |
| Example 2-6 | 1 /Tirzepatide | PLGA 504H | 9 | 1.50 |
| Example 2-7 | 1 /Tirzepatide | PLGA 504H | 9 | 3.00 |

### [Comparative Example 2] Preparation of microspheres including cationic salts other than calcium chloride

Microspheres were prepared in the same manner as in Example 1, except that sodium chloride (Comparative Examples 2-1 to 2-7 and Comparative Examples 2-10 to 2-12), zinc chloride (Comparative Example 2-8), or magnesium chloride (Comparative Example 2-9) was used as a cationic salt instead of calcium chloride.

Furthermore, microspheres were prepared in the same manner as in Example 2, except that sodium chloride (Comparative Example 2-13) was used as a cationic salt instead of calcium chloride.

The main components of the prepared microspheres are shown in Table 4.

**[Table 4]**

| Classification | W1 phase (mg) | O phase (mg) | | W2 phase (%(w/v)) | |
|---|---|---|---|---|---|
| | Amount / API | Type of polymer | Amount of polymer | Type of salt | Concentra tion |
| Comparative Example 1-1 | 1 /Semaglutide | PLGA 504H | 9 | - | - |
| Comparative Example 1-6 | 1 /Tirzepatide | PLGA 504H | 9 | - | - |
| Comparative Example 2-1 | 1 /Semaglutide | PLGA 504H | 9 | NaCl | 0.25 |
| Comparative Example 2-2 | 1 /Semaglutide | PLGA 504H | 9 | NaCl | 0.50 |
| Comparative Example 2-3 | 1 /Semaglutide | PLGA 504H | 9 | NaCl | 0.75 |
| Comparative Example 2-4 | 1 /Semaglutide | PLGA 504H | 9 | NaCl | 1.00 |
| Comparative Example 2-5 | 1 /Semaglutide | PLGA 504H | 9 | NaCl | 1.25 |
| Comparative Example 2-6 | 1 /Semaglutide | PLGA 504H | 9 | NaCl | 1.50 |
| Comparative Example 2-7 | 1 /Semaglutide | PLGA 504H | 9 | NaCl | 3.00 |
| Comparative Example 2-8 | 1 /Semaglutide | PLGA 504H | 6 | ZnCl₂ | 0.50 |
| Comparative Example 2-9 | 1 /Semaglutide | PLGA 504H | 6 | MgCl₂ | 0.50 |
| Comparative Example 2-10 | 1 /Semaglutide | PLGA 504H | 4 | NaCl | 0.25 |
| Comparative Example 2-11 | 1 /Semaglutide | PLGA 504H | 4 | NaCl | 0.50 |
| Comparative Example 2-12 | 1 /Semaglutide | PLGA 504H | 4 | NaCl | 0.75 |
| Comparative Example 2-13 | 1 /Tirzepatide | PLGA 504H | 9 | NaCl | 3.00 |

### [Example 3 and Comparative Example 3] Preparation of microspheres including plasticizer

A W1/O/W2 emulsion was prepared by adding a plasticizer to an oil phase layer solution, and microspheres were prepared in the same manner as in Example 1 or 2.

In Comparative Example 3-1, the prepared W1/O/W2 emulsion was heated above the glass transition temperature (Tg) of a biodegradable polymer (PLGA 504H) for 1 hour, and then maintained for 3 hours. In Comparative Example 3-2, a 100% polyvinyl alcohol (PVA) solution was added during vaporization; in Comparative Example 3-3, vaporization was carried out in a tank that was a vaporization tank with a baffle added; and in Comparative Example 3-4, 10% EtOH was added to the second water phase layer (W2) during vaporization. In Comparative Examples 3-5, 3-7, 3-8, and 3-10, 3.5% ethanol was added to the oil phase as a co-solvent, while for Comparative Examples 3-6 and 3-9, 0.6% Tween 80 was added to the oil phase as a surfactant.

In addition, in Comparative Example 3-10, 3.5% ethanol was added to the oil phase as a co-solvent, and microspheres were prepared in the same manner as in Example 2.

The main components of the microspheres prepared in Example 3 and Comparative Example 3 are shown in Tables 5 and 6.

**[Table 5]**

| Classific ation | W1 phase (g) | O phase (g) | | | | W2 phase (%(w/v)) | Added processes |
|---|---|---|---|---|---|---|---|
| | Amount / API | Type of polymer | Amoun t of polyme r | Solve nt (%) | Concentrat ion of surfactant added (%(w/v)) | Concentratio n of CaCl₂ or NaCl added | - |
| Compar ative Example 1-1 | 1 / Semaglutide | PLGA 504H | 9 | - | - | - | - |
| Example 1-6 | 1 / Semaglutide | PLGA 504H | 9 | - | - | 1.50% CaCl₂ | - |
| Example 1-7 | 1 / Semaglutide | PLGA 504H | 9 | - | - | 3.00% CaCl₂ | - |
| Compar ative Example 2-7 | 1 / Semaglutide | PLGA 504H | 9 | - | - | 3.00% NaCl | - |
| Example 1-14 | 1 / Semaglutide | PLGA 504H | 4 | - | - | 1.50% CaCl₂ | - |
| Compar ative Example 3-1 | 1 / Semaglutide | PLGA 504H | 9 | - | - | 1.50% CaCl₂ | Maintain vaporization temperature above Tg |
| Compar ative Example 3-2 | 1 / Semaglutide | PLGA 504H | 9 | - | - | 3.00% CaCl₂ | Add 100% water phase during vaporization |
| Compar ative Example 3-3 | 1 / Semaglutide | PLGA 504H | 9 | - | - | 3.00% CaCl₂ | Use tank equipped with baffle |
| Compar ative Example 3-4 | 1 / Semaglutide | PLGA 504H | 9 | - | - | 3.00% CaCl₂ | Apply water phase layer (W2) co-solvent (10% EtOH) |
| Compar ative Example 3-5 | 1 / Semaglutide | PLGA 504H | 9 | 3.5% EtOH | - | 3.00% CaCl₂ | - |
| Compar ative Example 3-6 | 1 / Semaglutide | PLGA 504H | 9 | - | 0.6% Tween 80 | 3.00% CaCl₂ | - |
| Compar ative Example 3-7 | 1 / Semaglutide | PLGA 504H | 9 | 3.5% EtOH | - | 3.00% NaCl | - |
| Compar ative Example 1-6 | 1 / Tirzepatide | PLGA 504H | 9 | - | - | - | - |
| Example 2-7 | 1 / Tirzepatide | PLGA 504H | 9 | - | - | 3.0% CaCl₂ | - |
| Compar ative Example 3-8 | 1 / Tirzepatide | PLGA 504H | 9 | 3.5% EtOH | - | 3.0% CaCl₂ | - |
| Compar ative Example 3-9 | 1 / Tirzepatide | PLGA 504H | 9 | - | 0.6% Tween 80 | 3.0% CaCl₂ | - |
| Compar ative Example 3-10 | 1 / Tirzepatide | PLGA 504H | 9 | 3.5% EtOH | - | - | - |

**[Table 6]**

| Classification | W1 phase (g) | O phase (g) | | | W2 phase (%(w/v)) |
|---|---|---|---|---|---|
| | Amount / API | Type of polymer | Amount of polymer | Concentration (%(w/v)) of plasticizer | Concentration of CaCl₂ or NaCl added |
| Example 3-1 | 1 / Semaglutide | PLGA 504H | 9 | 7.0% Triethylcitrate | 3.00% CaCl₂ |
| Example 3-2 | 1 / Semaglutide | PLGA 504H | 9 | 2.5% Triethylcitrate | 3.00% CaCl₂ |
| Example 3-3 | 1 / Semaglutide | PLGA 504H | 9 | 1.1% Triethylcitrate | 3.00% CaCl₂ |
| Example 3-4 | 1 / Semaglutide | PLGA 504H | 9 | 0.3% Poloxamer 188 | 3.00% CaCl₂ |
| Example 3-5 | 1 / Semaglutide | PLGA 504H | 9 | 0.3% Poloxamer 407 | 3.00% CaCl₂ |
| Example 3-6 | 1 / Semaglutide | PLGA 504H | 9 | 3.5% Triethylcitrate | 3.00% NaCl |
| Example 3-7 | 1 / Semaglutide | PLGA 504H | 9 | 3.0% Poloxamer 188 | 3.00% NaCl |
| Example 3-8 | 1 / Semaglutide | PLGA 504H | 9 | 0.3% Poloxamer 188 | 3.00% NaCl |
| Example 3-9 | 1 / Semaglutide | PLGA 504H | 4 | 3.5% Triethylcitrate | 1.50% CaCl₂ |
| Example 3-10 | 1 / Semaglutide | PLGA 504H | 4 | 2.5% Triethylcitrate | 1.50% CaCl₂ |
| Example 3-11 | 1 / Semaglutide | PLGA 504H | 4 | 1.1% Triethylcitrate | 1.50% CaCl₂ |
| Example 3-12 | 1 / Semaglutide | PLGA 504H | 4 | 0.3% Poloxamer 188 | 1.50% CaCl₂ |
| Example 3-13 | 1 / Semaglutide | PLGA 504H | 4 | 0.3% Poloxamer 407 | 1.50% CaCl₂ |
| Example 3-14 | 1 / Semaglutide | PLGA 504H | 9 | 2.5% Triethylcitrate | - |
| Example 3-15 | 1 / Semaglutide | PLGA 504H | 9 | 0.3% Poloxamer 188 | - |
| Example 3-16 | 1 / Tirzepatide | PLGA 504H | 9 | 2.5% Triethylcitrate | 3.00% CaCl₂ |
| Example 3-17 | 1 / Tirzepatide | PLGA 504H | 9 | 0.3% Poloxamer 188 | 3.00% CaCl₂ |
| Example 3-18 | 1 / Tirzepatide | PLGA 504H | 9 | 2.5% Triethylcitrate | - |
| Example 3-19 | 1 / Tirzepatide | PLGA 504H | 9 | 0.3% Poloxamer 188 | - |

### [Reference] Test method

### 1. Evaluation of content

5 mg of lyophilized microspheres of semaglutide or tirzepatide were put into a 100 mL volume flask, dissolved in 50 mL of acetonitrile, aligned with a marking line with D.W., and then filtered through a 0.45 syringe filter to quantify the amount of semaglutide or tirzepatide by high-performance liquid chromatography according to the following analysis conditions.

**[Table 7]**

| Semaglutide analysis conditions | |
|---|---|
| Column | Aegispak C8 (4.6 x 150 mm, 5.0 µm) |
| | Or a column of equivalent specifications |
| Mobile phase | MP A - 10 mM potassium dihydrogen phosphate solution (pH 2.0 by Phosphoric acid)MP B - Acetonitrile |
| Flow rate | 1 mL/min |
| Detector | UV, 215 nm |
| Gradient condition | Time (min)MP A (%)MP B (%)0802010307010.18020208020 |

| Tirzepatide analysis conditions | |
|---|---|
| Column | Aegispak C18 (4.6 x 250 mm, 5.0 µm) |
| | Or a column of equivalent specifications |
| Mobile phase | MP A - 0.1 % Trifluoroacetic Acid in WaterMP B - 0.1 % Trifluoroacetic Acid in Acetonitrile |
| Flow rate | 1 mL/min |
| Detector | UV, 220 nm |
| Gradient condition | Time (min)MP A (%)MP B (%)06040304555 |

### 2. Release test

10 mg of lyophilized microspheres of semaglutide or tirzepatide were dispersed in 10 mL of a release solution (prepared by dissolving 1.42 g of di-sodium hydrogen phosphate dihydrate and 14 g of propylene glycol in 5 L of water and adjusting the pH of the resulting solution to 7.4 with 1 N HCl), and a release test was performed using a Rotator (PTR-25) at 25 rpm and 37°C. The supernatant and microspheres were separated by centrifugation, and the amount of semaglutide or tirzepatide present in the supernatant was quantified by high-performance liquid chromatography. In this case, the analysis conditions were the same as those for the content analysis method.

### 3. SEM measurement

About 10 mg of microspheres was fixed on an aluminum stub, and platinum coating was performed under a vacuum degree of 0.1 torr and a high voltage (10 kV) for 3 minutes. Next, the microspheres were mounted on an SEM powder, and the surface of the microspheres was observed using an image analysis program.

### 4. Particle size measurement

The size of the microspheres was measured using a Mastersizer manufactured by Malvern Panalytical Ltd. About 100 mg of the microspheres was suspended in 5 ml of distilled water, the suspension was put into a dispersing device and dispersed under the conditions of 2,800 rpm and 50% ultrasound for 1 minute, and then the size was measured.

### 5. Osmotic pressure measurement

0.1 ml of the W2 layer was taken and introduced into a single-sample micro-osmometer manufactured by OsmoTech to measure the osmotic pressure.

### 6. Plasma analysis

The concentration analysis of a test substance in plasma was performed using LC-MS/MS at the analysis center of QuBEST BIO & KCRN Research. Plasma separation was performed at 12,000 rpm for about 2 minutes at 4°C. Pharmacokinetic analysis was performed by a non-compartmental method at the Nonclinical Evaluation Center of QuBEST BIO & KCRN Research using Phoenix WinNonlin software (Ver. 8.3, Certara). In this case, the method for analyzing the lC-Ms/MS of a plasma sample was as follows.

**[Table 8]**

| Analysis conditions | | | | |
|---|---|---|---|---|
| Column | ACE C18 (2.1 x 50 mm, 3.0 µm), ACE | | | |
| Column temperature | 40°C | | | |
| Mobile phase A | 10 mM ammonium acetate in distilled water | | | |
| Mobile phase B | 0.1% formic acid in acetonitrile | | | |
| Mobile phase conditions | Time (min) | Flow (ml/min) | A | B |
| | 0 | 0.4 | 55 | 45 |
| | 1.5 | 0.4 | 5 | 95 |
| | 2.5 | 0.4 | 5 | 95 |
| | 2.51 | 0.4 | 55 | 45 |
| | 4 | 0.4 | 55 | 45 |

### 7. Measurement of MC residual solvent

100 mg of the sample was dissolved in 1 mL of DMF, and the residual solvent was measured using Headspace-GC under the following analysis conditions.

**[Table 9]**

| Analysis conditions | |
|---|---|
| Column | DB-624, 0.53 mm × 30 m, 3 µm, Agilent Technologies |
| Column temperature | Maintain at 40°C for 5 minutes, then heat at 20°C per minute for 10 minutes to 240°C, and maintain at 240°C for 5 minutes |
| Flow rate | 1.2 mL/min |

| Headspace conditions | |
|---|---|
| Injection amount | 1 mL |
| Equilibrium temperature (°C) | 80 °C |
| Equilibrium time (min) | 30 min |

### 8. DSC measurement

DSC was measured using a differential scanning calorimeter. 5 to 10 mg of each of the microspheres was placed in an aluminum sample pan and measured at a rate of 5°C/min from 25°C to 250°C.

### 9. FTIR measurement

For FTIR measurement, a Nicolet iS5^{™} device manufactured by ThermoFisher Scientific was used. To utilize attenuated total reflectance (ATR), about 2 mg of the sample was applied onto a diamond laminate plate, and then analysis was performed using a single beam at a wavelength ranging from 600 to 4,000 cm⁻¹ and a resolution interval of 4 cm⁻¹.

### [Experimental Example 1] Confirmation of physical properties and initial burst control effects of deposit composition according to calcium chloride content

The following Tables 10 and 11 show the results of measuring the physical properties and initial burst effects of the depot compositions prepared in the examples and comparative examples.

**[Table 10]**

| Classification | W2 phase | Osmotic pressure (mOsm) | Residual solvent (ppm) | Day 1 (%) of long-term release |
|---|---|---|---|---|
| | Concentration of CaCl₂ (w/v %) | | | |
| Comparative Example 1-1 | 0.00 | 5 | 33.25 | 15.30 |
| Comparative Example 1-2 | 0.00 | 5 | N.D | 54.95 |
| Comparative Example 1-3 | 0.00 | 5 | N.D | 59.11 |
| Comparative Example 1-4 | 0.00 | 5 | N.D | 44.31 |
| Comparative Example 1-5 | 0.00 | 5 | N.D | 71.27 |
| Comparative Example 1-6 | 0.00 | 5 | 68.5 | 35.15 |
| Example 1-1 | 0.25 | 50 | N.D | 7.96 |
| Example 1-2 | 0.50 | 140 | N.D | 4.39 |
| Example 1-3 | 0.75 | 180 | N.D | 3.11 |
| Example 1-6 | 1.50 | 361 | 279.7 | 0.13 |
| Example 1-7 | 3.00 | 743 | 1183.00 | 0.00 |
| Example 1-8 | 0.25 | 50 | N.D | 9.08 |
| Example 1-9 | 0.50 | 140 | 2.60 | 6.00 |
| Example 1-10 | 0.75 | 180 | 21.61 | 2.69 |
| Example 1-11 | 0.25 | 50 | N.D | 35.79 |
| Example 1-13 | 0.75 | 180 | N.D | 28.72 |
| Example 1-14 | 1.50 | 361 | N.D | 18.55 |
| Example 1-15 | 0.50 | 140 | N.D | 29.39 |
| Example 1-16 | 0.50 | 140 | N.D | 24.50 |
| Example 1-17 | 0.50 | 140 | N.D | 49.07 |
| Example 1-18 | 0.50 | 140 | N.D | 34.58 |
| Example 1-19 | 0.50 | 140 | N.D | 31.42 |
| Example 1-20 | 0.50 | 140 | N.D | 19.22 |
| Example 2-1 | 0.50 | 140 | N.D | 48.53 |
| Example 2-2 | 1.50 | 361 | N.D | 10.39 |
| Example 2-3 | 0.50 | 140 | N.D | 26.41 |
| Example 2-4 | 1.50 | 361 | N.D | 8.43 |
| Example 2-5 | 0.50 | 140 | N.D | 6.22 |
| Example 2-6 | 1.50 | 361 | N.D | 0.00 |
| Example 2-7 | 3.00 | 701 | 483.8 | 0.00 |

**[Table 11]**

| Classification | W2 phase | Day 1 (%) of long-term release | Rat PK test Cₘₐₓ (ng/mL) |
|---|---|---|---|
| | Concentration of CaCl₂ (w/v %) | | |
| Comparative Example 1-1 | 0.00 | 15.30 | 1621.35 |
| Example 1-3 | 0.75 | 3.11 | 263.20 |
| Example 1-6 | 1.50 | 0.13 | 130.80 |
| Comparative Example 1-6 | 0.00 | 35.15 | 1814.05 |
| Example 2-6 | 1.50 | 0.00 | 183.50 |

As shown in Table 10, it can be confirmed that the initial burst of the depot composition is controlled when microspheres are prepared using calcium chloride. In addition, it can be confirmed that the initial burst (day 1 of long-term release) decreases in proportion to the amount of calcium chloride.

In Comparative Example 1-1, which did not include calcium chloride, the long-term release on day 1 was 15.3%, whereas in Example 1-6, which included 1.5% calcium chloride, the long-term release on day 1 was 0.13%, confirming that rapid initial burst was controlled. Through this, it can be confirmed that the initial burst reduction effects are present even when the ratio of drug to polymer varies (Examples 1-8 to 1-10 and 1-11 to 1-14).

Meanwhile, in Example 2-6, to which tirzepatide was applied, it can be confirmed that the initial burst reduction effect was similarly applied when compared with Comparative Example 1-6 (Comparative Example 1-6, Example 2-6).

Furthermore, it can be confirmed that the initial burst control effects are present even when the molecular weight of the polymer (PLGA) is different, as in Examples 1-15 and Comparative Example 1-3; when the type of polymer is different (change in the ratio of polylactic acid/polyglycolic acid in PLGA), as in Examples 1-16 and Comparative Example 1-4; and when a polylactide polymer (PLA) is used, as in Examples 1-17 and 1-18 and Comparative Example 1-5.

Further, as shown in Table 11, it can be confirmed that the maximum plasma concentration (Cmax) in animal experiments (administration of the test drug in rat PK tests) also decreases in proportion to the amount of calcium chloride.

As shown in the present invention, it can be confirmed that when calcium chloride at a concentration of 0 to 3.0% is used during the preparation of the depot composition, a depot composition with a low residual solvent can be prepared. However, when other cationic salts such as sodium chloride, magnesium chloride, or zinc chloride are used, it can be confirmed that the residual solvent has a high level of up to 2,200 ppm (Comparative Example 2-6). This could raise safety issues, in consideration of the permissible limits for residual solvents usable in pharmaceutical products.

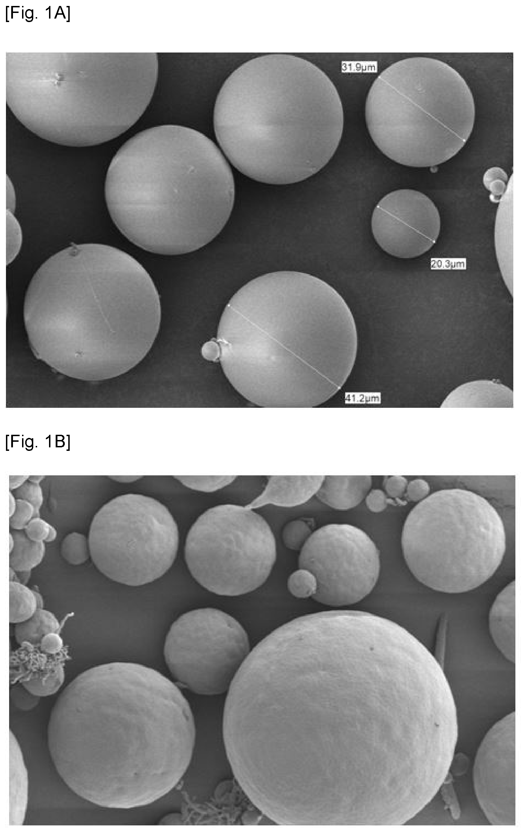

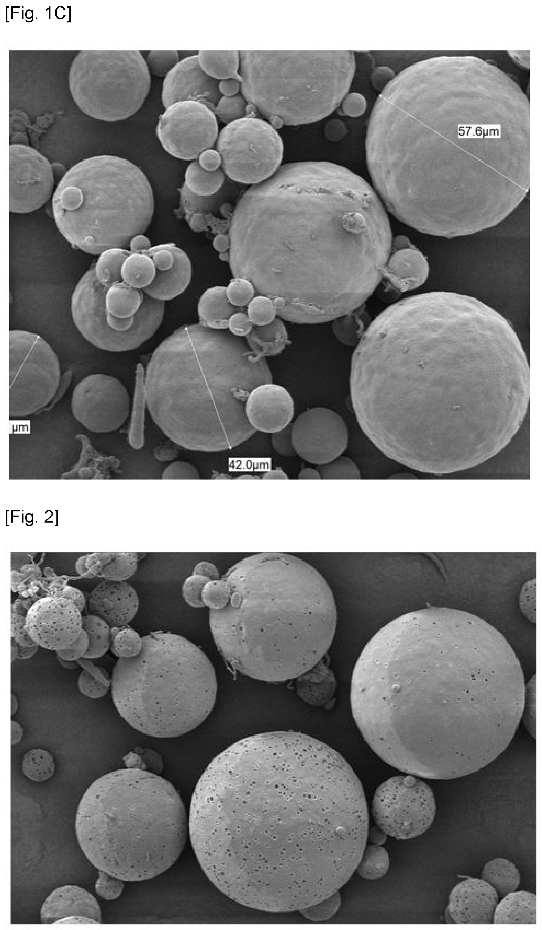

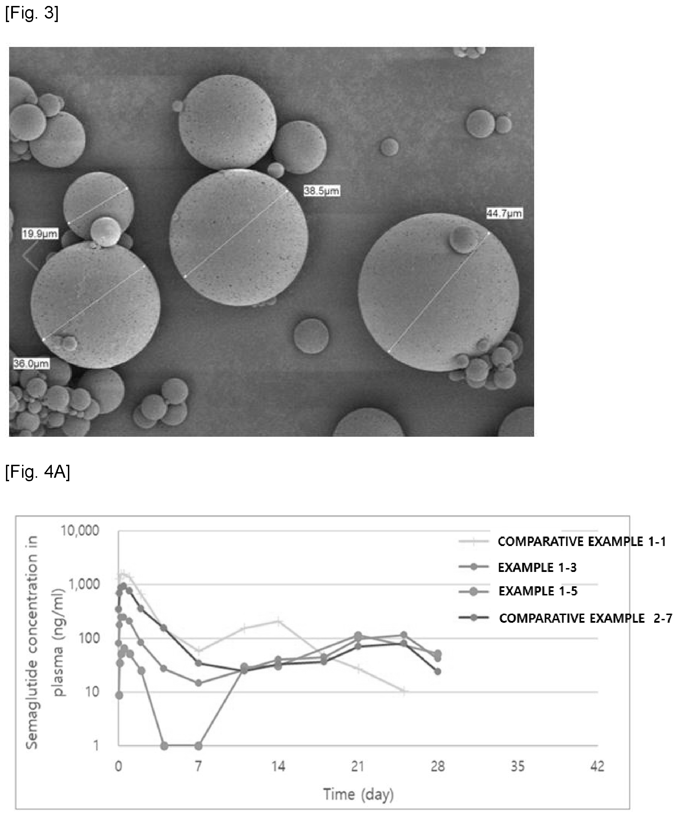

FIGS. 1A to 1C are SEM images of microspheres prepared according to Example 1, wherein FIG. 1A is an SEM image of microspheres prepared according to Example 1-3, FIG. 1B is an SEM image of microspheres prepared according to Example 1-9, and FIG. 1C is an SEM image of microspheres prepared according to Example 1-13. In addition, FIG. 2 shows an SEM image of microspheres prepared according to Comparative Example 1-1, and FIG. 3 shows an SEM image of microspheres prepared according to Comparative Example 2-7.

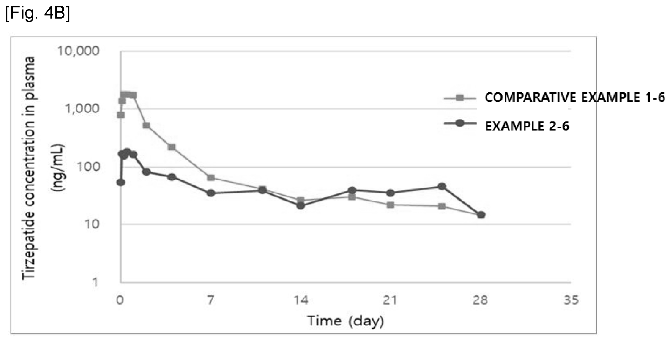

FIG. 4A shows the animal PK profiles of semaglutide microspheres prepared according to Comparative Example 1-1, Examples 1-3 and 1-5, and Comparative Example 2-7 of the present invention. Furthermore, FIG. 4B shows the animal PK profiles of tirzepatide microspheres prepared according to Comparative Example 1-6 and Example 2-6.

As shown in the figures above, it can be confirmed that Comparative Examples 1-1 and 2-7, in which calcium chloride was not used, have a large number of pores on the surface of the microspheres, and consequently, a large initial burst amount of API. It can be confirmed that the initial burst is similarly reduced not only in semaglutide but also in tirzepatide.

In general, microspheres are prepared in a homogenization process, and through the process of vaporizing the solvent used to dissolve the polymer, the solvent is scattered, and the microspheres are solidified. In the present invention, during the solidification process, calcium chloride may be brought into contact with the polymer on the surface of the microspheres, and a gelation phenomenon may occur at the interface due to hydrophobic ion pairing, thereby forming pore-free microspheres while coating the surface of the microspheres.

In particular, the case where calcium chloride is included in the W2 layer may have an effect of suppressing the initial burst of API while maintaining good properties. Preferably, when calcium chloride is included at a concentration of 0.25 to 3.0%, a depot composition with low residual solvent and suppressed initial burst may be prepared.

### [Experimental Example 2] Confirmation of physical properties and initial burst control effects according to type of cationic salt

The following Tables 12 and 13 show the results of measuring the physical properties and initial burst effects of the depot composition prepared in Comparative Example 2.

**[Table 12]**

| Classification | Type of salt | W2 phase | Osmotic pressure (mOsm) | Residual solvent (ppm) | Day 1 (%) of long-term release |
|---|---|---|---|---|---|
| | | Concentration of salt (w/v %) | | | |
| Comparative Example 1-1 | NaCl | 0.00 | 5 | 33.25 | 15.30 |
| Comparative Example 2-1 | NaCl | 0.25 | 92 | 1181.03 | 21.86 |
| Comparative Example 2-4 | NaCl | 1.00 | 300 | 1389.8 | 22.37 |
| Comparative Example 2-6 | NaCl | 1.50 | 568 | 2200.00 | 24.46 |
| Comparative Example 2-7 | NaCl | 3.00 | 1000 | 1506.80 | 22.85 |
| Comparative Example 2-8 | ZnCl2 | 0.50 | 151 | 1436.20 | 44.49 |
| Comparative Example 2-9 | MgCl2 | 0.50 | 165 | 1209.40 | 52.87 |
| Comparative Example 2-11 | NaCl | 0.50 | 181 | 1506.85 | 69.10 |
| Comparative Example 2-13 | NaCl | 3.00 | 1021 | 1906.4 | 29.10 |

**[Table 13]**

| Classification | W2 phase | Day 1 (%) of long-term release | Rat PK test Cmax (ng/mL) |
|---|---|---|---|
| | Concentration of NaCl (w/v %) | | |
| Comparative Example 1-1 | 0.00 | 15.30 | 1,621.35 |
| Comparative Example 2-7 | 3.00 | 22.85 | 1,145.75 |

As shown in Table 12, it can be confirmed that, when a depot composition is prepared using cationic salts other than calcium chloride, the amount of salt does not affect the control of the initial burst, unlike calcium chloride. Furthermore, as shown in Table 13, it can be confirmed that the initial burst control effect is also negligible in terms of the maximum plasma concentration (Cₘₐₓ) in animal experiments (administration of the test drug in rat PK tests). In addition, the residual solvent also exceeds the permissible limit (600 ppm or less of dichloromethane), making administration to humans impossible due to patient safety issues.

When sodium chloride is used as the cationic salt, hydrophobic ion pairing gelation does not occur on the surface of the fine particles, unlike calcium chloride, and therefore the initial burst control effect is thought to be very slight. Comparing Example 1-6 and Comparative Example 2-6, both at the same cationic salt concentration of 1.5%, the osmotic pressure of Example 1-6 was 361 mOsm, while that of Comparative Example 2-6 was 568 mOsm. Even though both had equivalent or higher osmotic pressures, the initial burst was evaluated as 0.13% and 24.46%, respectively. According to these results, it can be confirmed that the initial burst effect is independent of osmotic pressure. Furthermore, in Comparative Examples 2-6, osmotic pressure may cause high concentrations of the toxic solvent MC to remain, potentially leading to safety issues.

Further, when magnesium chloride or zinc chloride is used as the cationic salt, it can be confirmed that the properties of the microspheres are poor, the yield is low, and it is difficult to achieve formulation. Compared to the 6.00% result on day 1 of long-term release for Examples 1-9, the results on day 1 of long-term release for Comparative Examples 2-8 (using magnesium chloride) and Comparative Examples 2-9 (using zinc chloride) were 44.49% and 52.87%, respectively, confirming that the initial burst in the long-term release is not controlled even with other salts. That is, it can be confirmed that the corresponding cationic salt has a negligible effect in controlling the initial burst of the drug.

Through these test results, it can be confirmed that the initial burst control effect of the active ingredient can be obtained within the acceptable value of the residual solvent only when calcium chloride is used as the cationic salt as in the present invention.

Based on the osmotic pressure measurement results of the W2 layer in the examples and comparative examples, it is thought that the effect of calcium chloride on controlling the initial burst of API is not related to osmotic pressure. Furthermore, it can be confirmed that the W2 layer according to the cationic salts used in the examples and comparative examples was in the pH range of 5.6 to 5.8, indicating that there was no initial burst control effect due to pH changes. That is, it can be confirmed that the present invention has an effect of controlling the initial burst of API through gelation by hydrophobic ion pairing, which is a novel mechanism different from osmotic pressure or pH.

### [Experimental Example 3] Confirmation of physical property effects according to presence or absence and type of plasticizer used

The following Table 14 shows the results of measuring the physical properties of the depot compositions prepared in Example 3 and Comparative Example 3.

**[Table 14]**

| Classification | Amount of polymer | Type of salt | W2 phase Concentration of salt (w/v %) | Added processes | Oil phase Plasticizer/additive Concentration (w/v %) | Residual solvent (ppm) |
|---|---|---|---|---|---|---|
| Comparative Example 1-1 | 9 | CaCl₂ | 0.00 | - | - | 33.25 |
| Example 1-6 | 9 | CaCl₂ | 1.50 | - | - | 279.7 |
| Example 1-7 | 9 | CaCl₂ | 3.00 | - | - | 1,183.0 |
| Comparative Example 2-7 | 4 | NaCl | 3.00 | - | - | 1,506.8 |
| Example 1-14 | 4 | CaCl₂ | 1.50 | - | - | N.D |
| Comparative Example 3-1 | 9 | CaCl₂ | 1.50 | Maintain vaporization temperature above Tg | - | 98.0 |
| Comparative Example 3-2 | 9 | CaCl₂ | 3.00 | Add 100% water phase during vaporization | - | 1,198.0 |
| Comparative Example 3-3 | 9 | CaCl₂ | 3.00 | Use tank equipped with baffle | - | 1,184.0 |
| Comparative Example 3-4 | 9 | NaCl | 3.00 | Apply water phase layer (W2) co-solvent (10% EtOH) | - | 1,099.0 |
| Comparative Example 3-5 | 9 | CaCl₂ | 3.00 | - | 3.5% EtOH | N.D |
| Comparative Example 3-6 | 9 | CaCl₂ | 3.00 | - | 0.6% Tween 80 | 126.0 |
| Comparative Example 3-7 | 9 | NaCl | 3.00 | - | 3.5% EtOH | 117.0 |
| Comparative Example 1-6 | 9 | CaCl₂ | 0.00 | - | - | 68.5 |
| Example 2-7 | 9 | CaCl₂ | 3.00 | - | - | 483.8 |
| Comparative Example 3-8 | 9 | CaCl₂ | 3.00 | - | 3.5% EtOH | 228.1 |
| Comparative Example 3-9 | 9 | CaCl₂ | 0.00 | - | 0.6% Tween 80 | 55.8 |
| Comparative Example 3-10 | 9 | CaCl₂ | 0.00 | - | 3.5% EtOH | 70.5 |
| Example 3-1 | 9 | CaCl₂ | 3.00 | - | 3.5% Triethylcitrate | N.D |
| Example 3-2 | 9 | CaCl₂ | 3.00 | - | 2.5% Triethylcitrate | N.D |
| Example 3-3 | 9 | CaCl₂ | 3.00 | - | 1.1% Triethylcitrate | N.D |
| Example 3-4 | 9 | CaCl₂ | 3.00 | - | 3.0% Poloxamer 188 | N.D |
| Example 3-5 | 9 | CaCl₂ | 3.00 | - | 0.3% Poloxamer 188 | N.D |
| Example 3-6 | 9 | CaCl₂ | 3.00 | - | 0.3% Poloxamer 407 | N.D |
| Example 3-7 | 9 | NaCl | 3.00 | - | 3.5% Triethylcitrate | N.D |
| Example 3-8 | 9 | NaCl | 3.00 | - | 0.3% Poloxamer 188 | N.D |
| Example 3-9 | 4 | CaCl₂ | 1.50 | - | 3.5% Triethylcitrate | N.D |
| Example 3-10 | 4 | CaCl₂ | 1.50 | - | 2.5% Triethylcitrate | N.D |
| Example 3-11 | 4 | CaCl₂ | 1.50 | - | 1.1% Triethylcitrate | N.D |
| Example 3-12 | 4 | CaCl₂ | 1.50 | - | 0.3% Poloxamer 188 | N.D |
| Example 3-13 | 4 | CaCl₂ | 1.50 | - | 0.3% Poloxamer 407 | N.D |
| Example 3-14 | 9 | CaCl₂ | 0.00 | - | 2.5% Triethylcitrate | N.D |
| Example 3-15 | 9 | CaCl₂ | 0.00 | - | 0.3% Poloxamer 188 | N.D |
| Example 3-16 | 9 | CaCl₂ | 3.00 | - | 2.5% Triethylcitrate | N.D |
| Example 3-17 | 9 | CaCl₂ | 3.00 | - | 0.3% Poloxamer 188 | N.D |
| Example 3-18 | 9 | CaCl₂ | 0.00 | - | 2.5% Triethylcitrate | N.D |
| Example 3-19 | 9 | CaCl₂ | 0.00 | - | 0.3% Poloxamer 188 | N.D |

As shown in Table 14, it can be confirmed that when a depot composition is prepared using a plasticizer, the residual solvent is controlled according to whether or not a plasticizer is used and the type of plasticizer used. Through this, the safety issue of a patient may be resolved by significantly removing the residual solvent using a plasticizer.

Through this, it was confirmed that even when the cationic salt is changed into calcium chloride as well as sodium chloride, when calcium chloride is absent, or when the type of drug is changed (semaglutide and tirzepatide), the residual solvent can be remarkably reduced by applying a plasticizer.

When the vaporization temperature is adjusted to be above the glass transition temperature (Tg) of the biodegradable polymer (Comparative Example 3-1), the residual solvent decreases, but it is difficult to effectively reduce the residual solvent, and physiologically active substances may be denatured by heat. In addition, there is a disadvantage in that the properties of the microspheres deteriorate when the temperature is increased above the Tg temperature of a polymer. In the vaporization process, there is a method by which the residual solvent can be reduced by increasing the water phase (Comparative Example 3-2) or by installing a baffle in a vaporization tank (Comparative Example 3-3), but the results of this comparative example showed that the residual solvent is actually increased or it was difficult to remarkably reduce the residual solvent. Furthermore, it was confirmed that when a co-solvent was applied to the water phase layer (Comparative Example 3-4), it was difficult to remarkably reduce the residual solvent. Furthermore, even when the solvent EtOH was applied (Comparative Examples 3-5, 3-7, 3-8, and 3-10) or the surfactant Tween 80 was applied (Comparative Examples 3-6 and 3-9), it was difficult to reduce the residual solvent.

When the plasticizer according to the present invention is used and the corresponding amount of plasticizer is included in the oil phase (oil-based), the residual solvent level may be controlled to an N.D. level.

### [Experimental Example 4] Measurement of DSC and FTIR of depot compositions of examples and comparative examples

The following Table 15 shows the results of measuring the DSC and FT-IR of the depot compositions prepared in the examples and comparative examples.

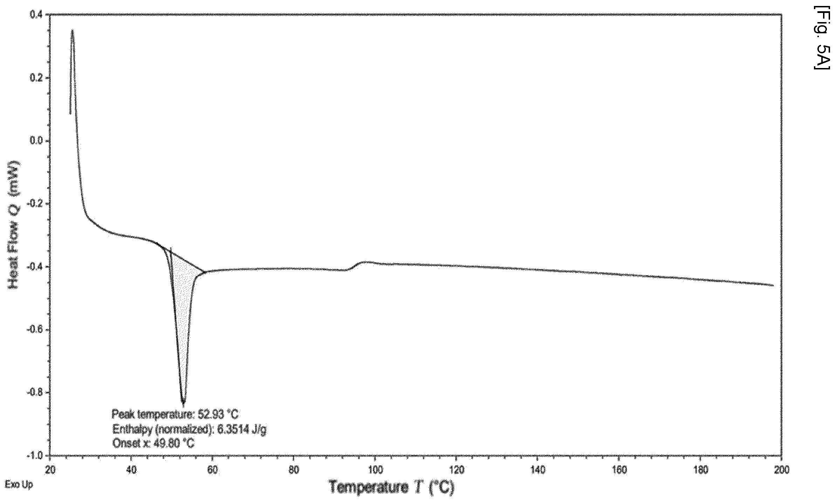

Furthermore, FIGS. 5A to 5E show the DSC results for 504H polymer (A) and microspheres prepared according to Comparative Examples 1-1 (B) and 2-7 (C) and Examples 1-3 (D) and 1-6 (E) of the present invention.

**[Table 15]**

| Classification | Type of salt | W2 phase Concentration of salt (w/v %) | Osmotic pressure (mOsm) | C=O FT-IR Wavenumber (cm⁻¹) | DSC Tg(°C) |
|---|---|---|---|---|---|
| 504H polymer | - | - | - | 1650.86 | 52.93 |
| | | | | 1537.56 | |
| Comparative Example 1-1 | - | 0.00 | 5 | 1651.57 | 51.62 |
| | | | | 1538.21 | |
| Comparative Example 2-7 | NaCl | 3.00 | 1000 | 1657.74 | 50.67 |
| | | | | 1546.96 | |
| Example 1-3 | CaCl₂ | 0.75 | 180 | 1657.72 | 53.60 |
| | | | | 1543.63 | |
| Example 1-6 | CaCl₂ | 1.50 | 361 | 1660.21 | 52.20 |
| | | | | 1539.50 | |

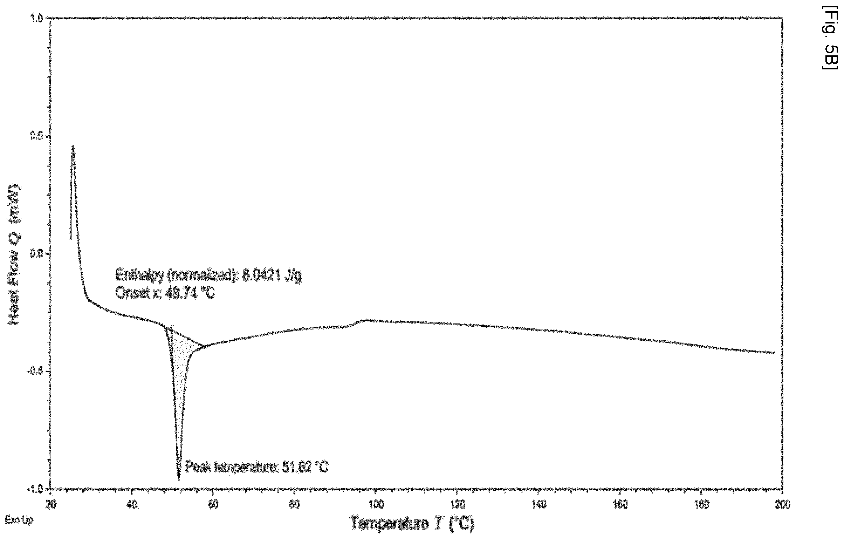

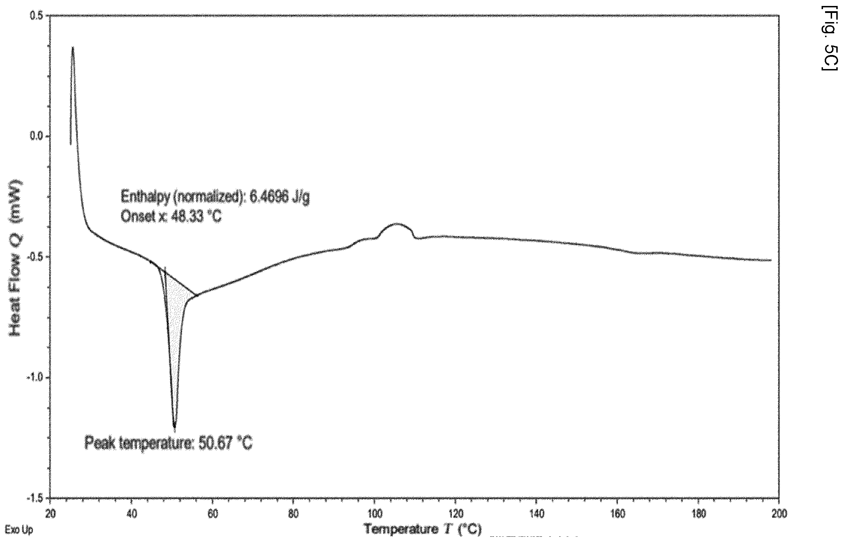

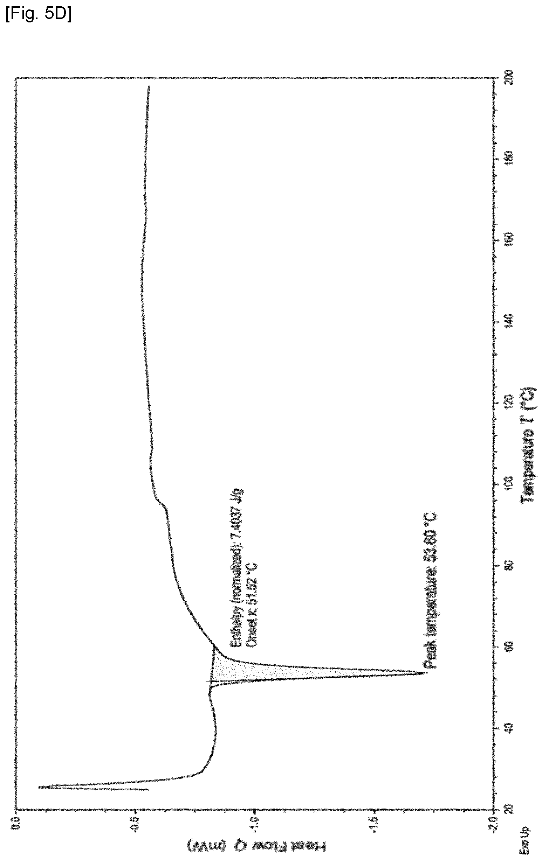

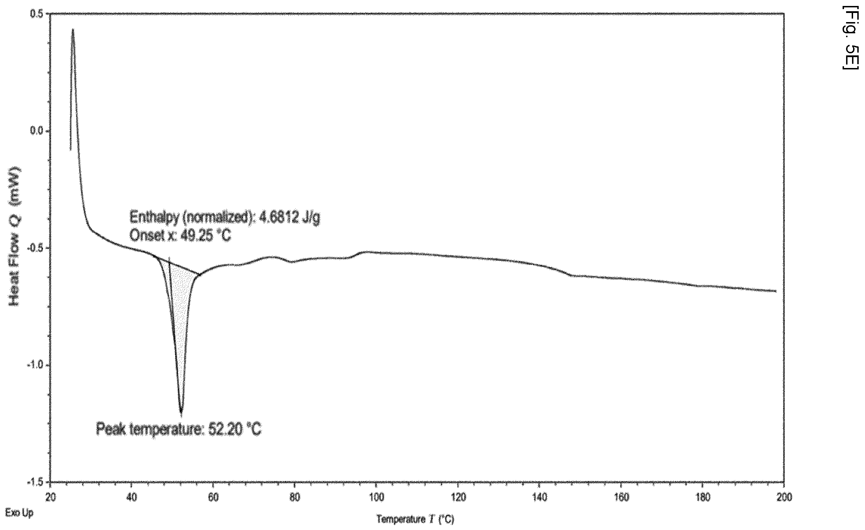

As shown in Table 15 and FIG. 5 described above, when the measurement of the polymer alone (FIG. 5A) is compared to the measurement without the application of a cationic salt (Comparative Example 1-1, FIG. 5B), there was a negligible change in the Tg value. When calcium chloride was used ((Example 1-3, FIG. 5D), (Example 1-6, FIG. 5E)), the Tg value increased slightly, but the difference was negligible, and when sodium chloride was used (Comparative Example 2-7, FIG. 5C), the Tg value decreased slightly, but the difference was negligible.

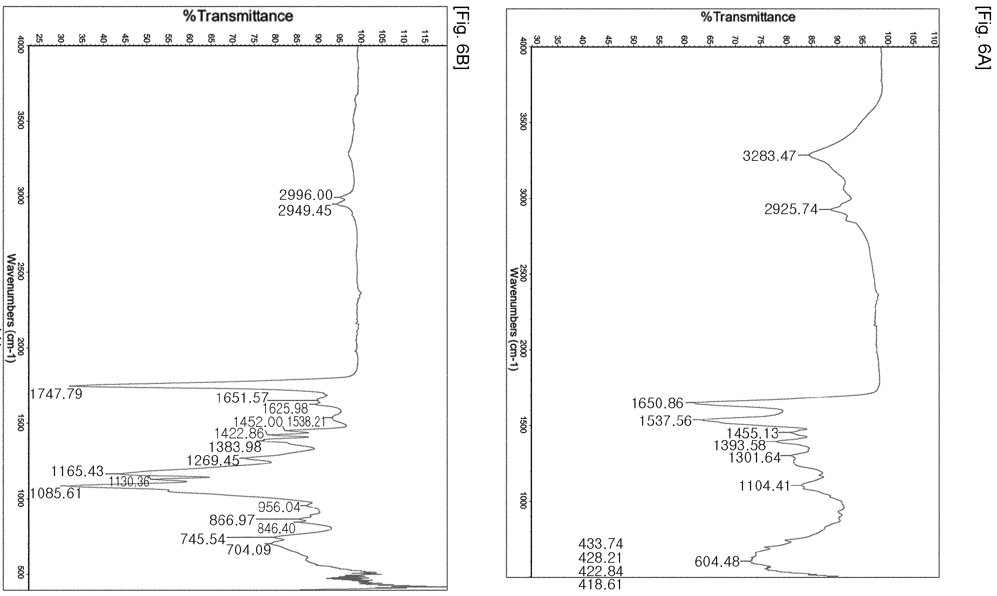

Furthermore, FIGS. 6A to 6E show the FT-IR results for 504H polymer (A) and microspheres prepared according to Comparative Examples 1-1 (B) and 2-7 (C) and Examples 1-3 (D) and 1-6 (E) of the present invention.

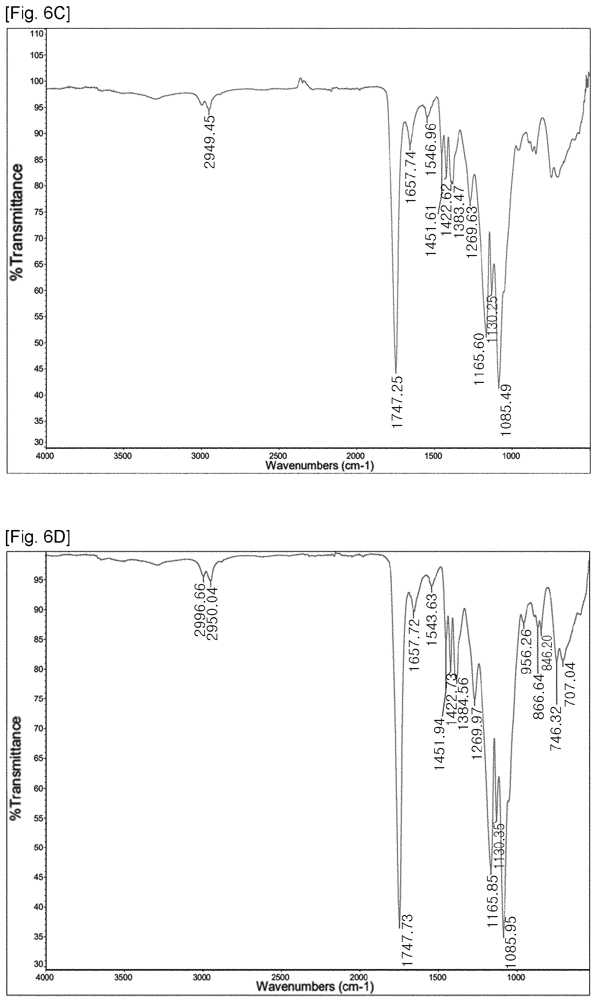

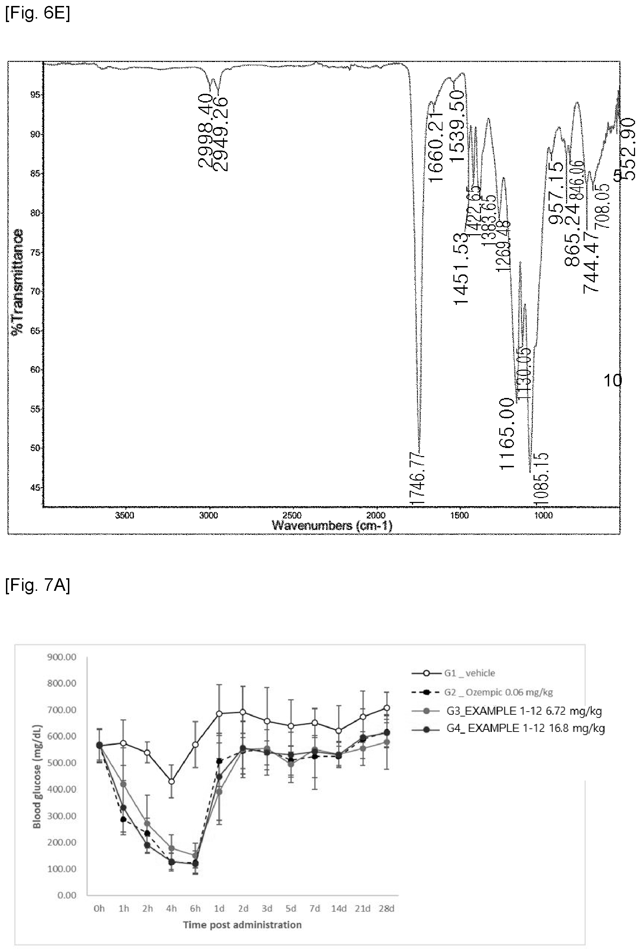

In FIG. 6, when a polymer alone was measured (FIG. 6A) and when a cationic salt was not used (Comparative Example 1, FIG 6B), a change was confirmed near the FT-IR peak of the carboxyl group of a polymer, and when a cationic salt was used, a change in the peak near the C=O peak was confirmed. In particular, when calcium chloride was used ((Examples 1-3, FIG. 6D), (Examples 1-6, FIG. 6E)), as the concentration increased, the peak of 1650.86 cm⁻¹ in Comparative Example 1-1 shifted to 1657.72 cm⁻¹ and 1660.21 cm⁻¹, respectively, and the peak of 1537.56 cm⁻¹ shifted to 1543.63 cm⁻¹ and 1539.50 cm⁻¹, respectively. In the case of sodium chloride, which has a negligible effect in the initial burst (Comparative Example 2-7, FIG. 6C), the peaks shifted to 1657.74 cm⁻¹ and 1546.96 cm⁻¹, thus confirming a negligible change in the peaks even when high concentrations were applied.

To summarize the results above, when calcium chloride is applied, the DSC results remain unchanged, and a shift in the peak is confirmed in FT-IR. This means that gelation occurs due to a physical phenomenon on the polymer surface due to hydrophobic ion pairing by calcium chloride and a coating effect resulting therefrom appears.

### [Experimental Example 5] Measurement of blood glucose and glycated hemoglobin (HbA1c)

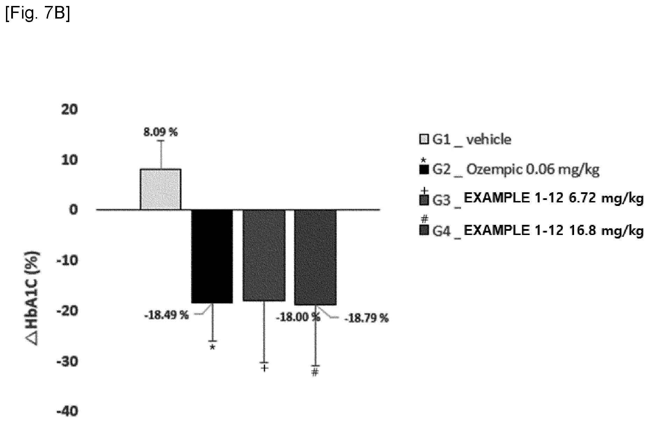

After 5- to 6-week-old diabetic db/db mice were obtained, they underwent an acclimatization period, and then had access to food and water ad libitum from day 0. The animals were divided into groups based on body weight, and their body weight and feed intake were measured once every two days until the end of the experiment. After administering the medication while avoiding fasting, blood glucose levels were measured a total of 13 times over 0 (before administration), 1 hour, 2 hours, 4 hours, 6 hours, 1 day, 2 days, 3 days, 5 days, 7 days, 14 days, 21 days, and 28 days, and the measurement results are shown in FIGS. 7A and 7B.

In FIG. 7, G1 vehicle shows the case where no experimental substance was administered, G2 vehicle shows the control drug (Ozempic) administered daily at 0.06 MPK for 4 weeks, G3 vehicle shows the semaglutide depot composition according to the present invention (Examples 1-12) administered once at 6.72 mpk, and G4 vehicle shows the semaglutide depot composition according to the present invention (Examples 1-12) administered once at 16.8 mpk.

As shown in FIG. 7A, it can be confirmed that the G3 and G4 groups, in which the depot composition according to the present invention is used, have a blood glucose lowering effect equivalent to or greater than that of the control drug. As a result of confirming blood glucose levels for 4 weeks after administration, after 4 weeks without any side effects of a rapid blood glucose lowering phenomenon, the G2 (control drug) group and the G3 and G4 groups showed a reduction in blood glucose of 12.9%, 18.4% and 13.5%, respectively, compared to the G1 (vehicle) group.

Furthermore, as shown in FIG. 7B, it can be confirmed that the G3 and G4 groups, in which the depot composition according to the present invention is used, have a glycated hemoglobin lowering effect equivalent to or greater than that of the control drug. Comparing the results 4 weeks after administration with those before administration, the G3 and G4 groups showed a reduction by -18.00% and -18.79%, respectively, while the control group secured similar results as the glycated hemoglobin was reduced by -18.49%.

### [Experiment Example 6] Body weight measurement

From the time when 7-week-old mice were obtained (day 0), the animals had access to a 60% kal% high-fat diet food ad libitum for 22 weeks, except for the control group, and their individual body weight and feed intake were measured twice a week until autopsy. From the starting week of the test until the week before dissection, their body weight and feed intake were measured by dividing the daily food intake per cage by the number of animals. At 18 weeks of induction, the animals were divided into groups based on body weight, and after subcutaneous administration of the drug from Example 1-12, blood glucose levels were measured weekly for four weeks. To minimize errors during the measurement, their body weight and food intake were measured at the same time period on the same day using the same balance throughout the study period.

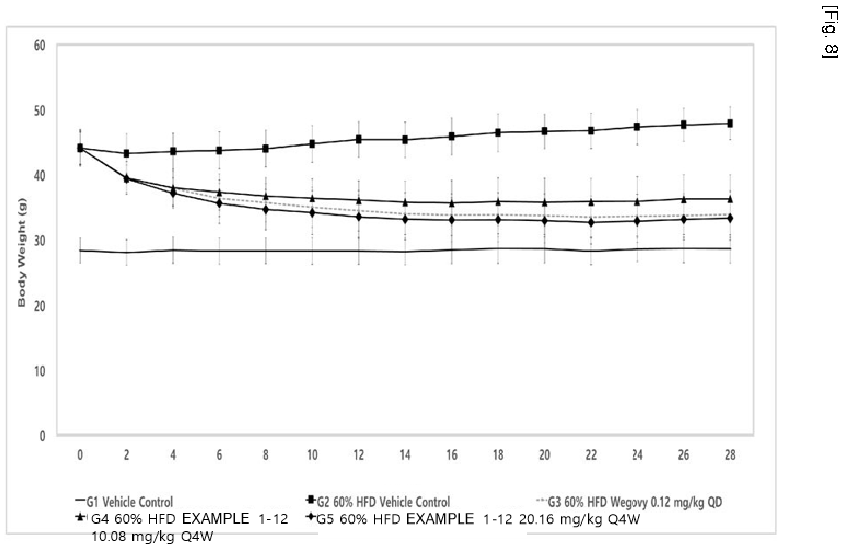

The measurement results are shown in FIG. 8. In FIG. 8, G1 is a group consisting of typical control mice that did not receive a high-fat diet (negative control group), while G2 vehicle is a group consisting of mice that received a high-fat diet, but were not administered the experimental substance. G3 represents a group in which the control drug is administered daily at 0.12 mpk, G4 represents a group in which the semaglutide depot composition (Example 1-12) is administered once at 10.08 mpk, and G5 represents a group in which the semaglutide depot composition (Example 1-12) is administered once at 20.16 mpk.

As shown in FIG. 8, body weight lowering effects equivalent to or greater than those of existing drugs can be confirmed at 10.08 mpk and 20.16 mpk. (G3 group: reduction by 29.2% compared to vehicle group, G4 group: reduction by 23.0% compared to vehicle group, and G5 group: reduction by 29.4% compared to vehicle group)

### [Industrial Applicability]

The microspheres of the depot composition of the present invention can control excessive release of an active ingredient contained inside the microspheres at the initial stage of injection of a depot preparation by including calcium chloride in the water phase layer. In addition, the composition has excellent suspendability and a significantly small amount of residual solvent, and thus even when administered as an injection by a user, the effect of the active ingredient can be uniformly, safely, and continuously obtained.

In addition, improved medical therapy with semaglutide and/or tirzepatide can be provided by adjusting the dose of semaglutide and/or tirzepatide by means of the depot formulation.

## Claims

1. A pharmaceutical composition for use in preventing or treating obesity or diabetes, comprising semaglutide or a pharmaceutically acceptable salt thereof; or tirzepatide or a pharmaceutically acceptable salt thereof, as an active ingredient,
wherein the dosage of a GLP-1 receptor agonist for preventing or treating obesity is 12 to 96 mg/4 weeks, and
the dosage of a GLP-1 receptor agonist for preventing or treating diabetes is 4 to 80 mg/4 weeks.

2. The pharmaceutical composition of claim 1, wherein the dosage of semaglutide or a pharmaceutically acceptable salt thereof; or tirzepatide or a pharmaceutically acceptable salt thereof, for preventing or treating obesity, is 28 to 48 mg/4 weeks.

3. The pharmaceutical composition of claim 1, wherein the dosage of semaglutide or a pharmaceutically acceptable salt thereof; or tirzepatide or a pharmaceutically acceptable salt thereof, for preventing or treating diabetes, is 12 to 32 mg/4 weeks.

4. The pharmaceutical composition of claim 1, wherein semaglutide or a pharmaceutically acceptable salt thereof; or tirzepatide or a pharmaceutically acceptable salt thereof is administered to a subject as a microneedle formulation or depot formulation.

5. The pharmaceutical composition of claim 4, wherein semaglutide or a pharmaceutically acceptable salt thereof; or tirzepatide or a pharmaceutically acceptable salt thereof is included in an amount of 5 to 25 parts by weight based on 100 parts by weight of each formulation.

6. The pharmaceutical composition of claim 1, wherein, when the pharmaceutical composition is administered to a subject, semaglutide or a pharmaceutically acceptable salt thereof, or tirzepatide or a pharmaceutically acceptable salt thereof has an AUCt/Cₘₐₓ of 100 hours or more.

7. A depot composition comprising microspheres in an O/W2 form, comprising an oil phase layer (O layer) and a second water phase layer (W2 layer), or microspheres in a W1/O/W2 form, comprising a first water phase layer (W1 layer), an oil phase layer (O layer), and a second water phase layer (W2 layer),
wherein the oil phase layer (O layer) in the microspheres in the O/W2 form or the first water phase layer (W1 layer) in the microspheres in the W1/O/W2 form comprises semaglutide or a pharmaceutically acceptable salt thereof; or tirzepatide or a pharmaceutically acceptable salt thereof, and
in the microspheres in the O/W2 form or microspheres in the W1/O/W2 form, the oil phase layer (O layer) comprises a biodegradable polymer, and the second water phase layer (W2 layer) comprises calcium chloride.

8. The depot composition of claim 7, wherein the biodegradable polymer comprises a polymer selected from the group consisting of polylactide (PLA), polyglycolide (PGA), and polylactide-co-glycolide (PLGA).

9. The depot composition of claim 7, wherein calcium chloride is included at a concentration of 0.1 to 5.0% (w/v) based on the second water phase layer (W2 layer).

10. The depot composition of claim 7, wherein the oil phase layer (O layer) in the microspheres in the O/W2 form or in the microspheres in the W1/O/W2 form further comprises a plasticizer.

11. The depot composition of claim 10, wherein the plasticizer is one or more selected from the group consisting of glycerin (glycerol), concentrated glycerin, sorbitol, propylene glycol, a poloxamer-based polymer, a phthalate polymer, a citrate derivative, triacetin, and castor oil.

12. A method for preparing a depot composition, the method comprising: preparing an oil phase layer (O layer) solution or mixing a first water phase layer (Water phase 1: W1 layer) solution with the oil phase layer (O layer) solution to prepare a W1/O emulsion;
introducing the oil phase layer (O layer) solution or W1/O emulsion into a second water phase (Water phase 2: W2 layer) layer solution to prepare an O/W2 emulsion or W1/O/W2 emulsion,
wherein the oil phase layer (O layer) solution in the O/W2 emulsion, or the water phase layer (W1 layer) solution in the W1/O/W2 emulsion comprises semaglutide or a pharmaceutically acceptable salt thereof; or tirzepatide or a pharmaceutically acceptable salt thereof, and
the oil phase layer (O layer) solution in the O/W2 emulsion or W1/O/W2 emulsion comprises a biodegradable polymer, and the second water phase layer (W2 layer) solution comprises calcium chloride.

13. The method of claim 12, wherein calcium chloride is included at a concentration of 0.1 to 5.0% (w/v) based on the second water phase layer (W2 layer) solution.

14. The method of claim 12, wherein the oil phase layer (O layer) in the microspheres in the O/W2 form or in the microspheres in the W1/O/W2 form further comprises a plasticizer.

15. The method of claim 12, further comprising recovering microspheres by drying and/or filtering the prepared O/W2 emulsion or W1/O/W2 emulsion and then centrifuging the emulsion.
